(19) Europäisches Patentamt

European Patent Office

Office européen des brevets

(11) Numéro de publication: **0 271 379**
A1

(12) # DEMANDE DE BREVET EUROPEEN

(21) Numéro de dépôt: 87402539.8

(22) Date de dépôt: 10.11.87

(51) Int. Cl.4: **C 12 P 21/00**
C 12 N 5/00, C 12 N 15/00,
A 61 K 39/40
//(C12P21/00,C12R1:91)

(30) Priorité: 12.11.86 FR 8615671

(43) Date de publication de la demande:
15.06.88 Bulletin 88/24

(84) Etats contractants désignés:
AT BE CH DE ES FR GB GR IT LI LU NL SE

(71) Demandeur: ROUSSEL-UCLAF
35, boulevard des Invalides
F-75007 Paris (FR)

(72) Inventeur: Smets, Pierre
137, Avenue du Maréchal Leclerc
F-78670 Villennes Sur Seine (FR)

Salles, Marie-France
97, Avenue de Villiers
F-75017 Paris (FR)

Zalisz, René
20, rue Delattre de Tassigny
F-95180 Menucourt (FR)

(74) Mandataire: Vieillefosse, Jean-Claude et al
Département des Brevets ROUSSEL UCLAF B.P no 9
F-93230 Romainville (FR)

(54) Anticorps monoclonaux anti-gram (-), procédé de préparation et applications.

(57) Procédé de préparation d'anticorps monoclonaux de mammifères dirigés contre la partie lipide A-core de l'endotoxine des bactéries gram (-) de différents genres, caractérisé en ce que l'antigène servant à l'immunisation des cellules immunocompétentes est un lipopolysaccharide complet de bactérie gram (-) dont la partie lipide A-core est extériorisée.

EP 0 271 379 A1

**Description**

Anticorps monoclonaux antigram (-), procédé de préparation et applications.

Des enquêtes réalisées aux USA ont montré que les septicémies à bactéries gram (-) sont en augmentation constante. Une étude clinique réalisée par Ziegler en 1982 a mis en évidence l'amélioration thérapeutique apportée par la sérothérapie dans le traitement de ces septicémies à gram (-) et des chocs endotoxiniques.

Les anticorps monoclonaux (AcM) pourraient se substituer à la sérothérapie dans le traitement de ces affections.

L'endotoxine des bactéries gram (-) est un lipopolysaccharide antigénique (LPS).

Les anticorps dirigés contre la partie antigène 0 (ou 0-side chain) du LPS sont spécifiques à la fois de l'espèce et du type de la bactérie. Par contre, les anticorps dirigés contre la région lipide A-core de l'endotoxine de la bactérie sont susceptibles de réagir avec différents genres de bactéries gram (-). En effet, la partie lipide A-core est relativement constante chez les bactéries gram (-).

Un certain nombre de références ont décrit des anticorps monoclonaux capables de réagir avec des bactéries gram (-) de différents genres.

On peut citer les demandes de brevet PCT n☒ WO 84/04458 et WO 85/01659 ainsi que la demande de brevet européen n☒ 174.204.

La demande PCT WO 84/04458 utilise comme antigène l'endotoxine de mutants "rough" ayant perdu l'antigène 0 et éventuellement une partie des sucres du core pour obtenir cette absence de spécificité. Elle précise également qu'il est possible d'utiliser des endotoxines complètes que l'on dégrade partiellement pour obtenir une structure simplifiée de l'antigène, du genre de l'endotoxine des mutants "rough".

En effet, les LPS comportent un pôle hydrophobe du côté du lipide A et un pôle hydrophile du côté de l'antigène 0. Dans un milieu aqueux, ces LPS s'associent en structure micellaire, avec la partie lipide A au centre et l'antigène 0 vers l'extérieur des micelles.

La partie antigénique commune aux différents genres est donc cachée et n'étant pas exposée, ne peut pas générer d'anticorps non spécifiques. C'est pourquoi un LPS incomplet est nécessaire.

Cette même approche a été adoptée dans la demande WO 85/01659 qui utilise comme antigènes des endotoxines de type LPS de mutants "rough". Cette demande décrit notamment un mélange de différents anticorps monoclonaux murins protégeant contre le choc endotoxinique provoqué par Escherichia Coli J5 (Ec J5).

Contrairement à l'idée reçue, la demanderesse a obtenu des AcM réagissant avec des bactéries gram (-) de différents genres, bien qu'en utilisant comme antigène une endotoxine complète de bactéries "smooth" donc comportant leur partie antigène 0.

C'est pourquoi, la présente demande a pour objet un procédé de préparation d'anticorps monoclonaux de mammifères dirigés contre la partie lipide A-core des endotoxines des bactéries gram (-) de différents genres caractérisé en ce que l'antigène servant à l'immunisation de la cellule immunocompétente est un lipopolysaccharide complet d'une bactérie gram (-) dont la partie lipide A-core est extériorisée.

Par LPS complet, on entend l'endotoxine comprenant la partie lipide A, la partie core et toute ou la majeure partie antigène 0.

Lorsque l'on dit que la partie lipide A-core du LPS est extériorisée, l'on entend que la partie lipide A-core du LPS normalement non exposée au contact avec les cellules immunocompétentes, se trouve exposée et possède donc la capacité de stimuler les lymphocytes B.

Le LPS peut provenir de n'importe quelle espèce de bactérie gram (-) mais de préférence de bactéries des genres Klebsiella, Escherichia, Pseudomonas, Enterobacter, Bacteroïdes, Serratia ou Proteus et notamment du genre Klebsiella, particulièrement de Klebsiella pneumoniae ainsi que d'Escherichia coli.

Dans des conditions préférentielles de mise en oeuvre du procédé ci-dessus décrit, le LPS a une structure LPS-LAP. Par LAP, on entend : Protéines associées au lipide A (Morrison : The journal of Immunology Vol.119 n☒5 Nov. 1977, p.1790 et Handbook of Endotoxin Vol.1 Chemistry of Endotoxin, Ed. RIETSCHEL ELSEVIER (1984) p.339 et suivantes : the protein component of bacterial endotoxins).

Les LPS - LAP peuvent être préparés selon les méthodes décrites dans la littérature. Les LPS - LAP préférés sont ceux dont la préparation est décrite dans les brevets européens n☒ 49182 et 115988 dans lesquels ils ont été nommés glycoprotéines.

Ils peuvent notamment être préparés par culture des microorganismes jusqu'à développement complet, lyse des germes de préférence enzymatique puis chimique, séchage du lysat, extraction à l'aide de solvants des lipides, de préférence à l'acétone puis au méthanol, mise en suspension du lysat dans de l'eau, déprotidation physique, de préférence par centrifugation, ultrafiltration de la solution sous pression, de préférence à l'aide d'un ultrafiltre à seuil de coupure de 300.000, précipitation à l'aide d'un halogénure d'ammonium quaternaire, concentration du surnageant de préférence à l'aide d'ultrafiltres à seuil de coupure de 5000 à 100.000 et précipitation des LPS - LAP recherchés à l'aide d'un alcanol de faible poids moléculaire.

Les procédés in vivo et in vitro de préparation d'anticorps monoclonaux sont connus dans la littérature, notamment par les brevets PCT WO 84/04458 et 85/01659 précités.

Contrairement aux enseignements de l'art antérieur, l'immunisation est opérée à l'aide d'un LPS complet, mais dont la partie lipide A-core est extériorisée. Par exemple, des souris recevront par voie parentérale (sous cutanée, intra-musculaire ou intrapéritonéale) une ou plusieurs injections de LPS - LAP à la dose préférentielle de 1 mg par kg en présence ou non d'un adjuvant tel que l'adjuvant de Freund. Une dernière injection est faite par voie intraveineuse trois jours avant le prélèvement des cellules spléniques en vue de l'hybridation.

Les autres étapes : fusion avec cellules de myélome, détection des hybridomes secrétant les anticorps dirigés contre l'antigène d'immunisation, conservation des hybridomes, clonage, production des anticorps sont réalisés selon les méthodes connues.

La présente demande a également pour objet les AcM obtenus par le procédé ci-dessus décrit, notamment d'origine murine ou humaine, ainsi que les hybridomes secréteurs desdits anticorps, notamment ceux désignés par $D_6 B_4$, $6 D_5$, $12 B_6$, $B_7B_3$, $E_{22}B_6$ et $H_7A_2$.

Ces anticorps monoclonaux trouvent leur utilisation en thérapeutique à des fins curatives ou préventives, chez l'homme ou l'animal. C'est pourquoi la présente demande a aussi pour objet les médicaments constitués par lesdits anticorps monoclonaux, de même que les compositions pharmaceutiques renfermant lesdits AcM. Elle vise aussi l'utilisation desdits anticorps monoclonaux dans une méthode de traitement du corps humain ou animal, ainsi que leur utilisation en vue de la préparation de médicaments destinés au corps humain ou animal.

Ces anticorps monoclonaux trouvent aussi leur utilisation à des fins diagnostiques, pour la détection, l'identification ou le dosage des bactéries gram (-), de l'endotoxine des bactéries gram (-) ou de la partie lipide A-core de l'endotoxine des bactéries gram (-). C'est pourquoi, la présente demande a également pour objet l'utilisation desdits anticorps ainsi que les compositions diagnostiques destinées à la détection, l'identification ou le dosage des antigènes ci-déssus.

Ces anticorps monoclonaux trouvent également leur utilisation dans la purification de produits renfermant la partie antigénique aspécifique des bactéries gram (-). La présente demande a donc également pour objet l'utilisation desdits AcM pour la purification, ainsi que les compositions destinées à la purification de produits renfermant la partie antigénique aspécifique des bactéries gram (-) caractérisées en ce qu'elles utilisent lesdits anticorps monoclonaux.

Les lignées cellulaires hybridomes suivantes $6 D_5$, $12 B_6$, $D_6B_4$ ont été déposées le 5 novembre 1986 auprès de la Collection Nationale de Culture de Microorganismes (CNCM - Institut Pasteur) à Paris sous les n⚊ I 618, I 619 et I 620.

Les lignes cellulaires hybridomes suivantes $B_7B_3$ (anti E. Coli, réagissant aussi avec Serratia et Pseudomonas aeruginosa), $E_{22}B_6$ (anti-Proteus) et $H_7A_2$ (anti-Enterobacter) ont également été déposées en date du 9 Novembre 1987 auprès de la Collection Nationale de Culture de Microorganismes (CNCM-Institut Pasteur) à Paris sous le n⚊ I - 711, I - 712 et I - 713 .

La souche de Klebsiella pneumoniae CIP 52145 a été déposée par la demanderesse à la CNCM sous le n⚊ I 163 en date du 25 Juin 1981.

La souche d'Escherichia coli 0111 $B_4$ est bien connue, notamment son mutant J5 et a été déposée par diverses sociétés auprès de différentes collections de Microorganismes.

Les exemples qui suivent illustrent la présente invention sans toutefois la limiter.

Exemple 1

I. Préparation des antigènes

1) Préparation de la Biomasse.

La culture est réalisée en fermenteur de 15 litres dans un milieu :
Bouillon Trypto - Caséine Soja
Hydrolysat tryptique de Caséine   17 g/l
Peptone papaïnique de soja   3 g/l
Chlorure de sodium   5 g/l
Phosphate bipotassique   2,5 g/l
Dextrose   2,5 g/l.
en ajustant le pH à environ 7,3 après ensemencement à l'aide de la souche lyophilisée conservée sur milieu gélosé remise en suspension dans du liquide physiologique.

2) Récupération des germes.

Au terme de 15 heures de culture, les germes sont centrifugés 30 minutes à 11.500 g, lavés 2 fois par une solution apyrogène de NaCl à 9 ⚊/⚊ ⚊ puis repris dans un litre d'eau distillée stérile apyrogène.

3) Lyse.

Les germes sont alors chauffés à 70⚊C durant 1 heure et demie. Après refroidissement à température ambiante, la lyse est réalisée par adjonction de 1,25 g d'acide éthylène diamine tétracétique, d'1 g de mercurothiolate de sodium et 8 ml de lysozyme de blanc d'oeuf (100 g/litre).

La solution est laissée à température ambiante durant 8 jours puis lyophilisée.

4) Préparation de l'antigène.

5 litres d'acétone sont ensuite ajoutés à la poudre bactérienne et agités durant 1 heure et demie. Après décantation, la poudre est filtrée sur verre fritté type G2 et séchée à 40⚊C 16 heures sous pression réduite.

La poudre est ensuite traitée par 5 litres de méthanol durant 1 heure et demie sous agitation. Le résidu est filtré et séché comme précédemment.

Les germes ainsi traités sont alors repris dans de l'eau (20 g/litre) et maintenus sous agitation pendant 16 heures à +4≠C.

La suspension est alors centrifugée 45 minutes à 11.500 g et le surnageant est purifié par ultrafiltration sur membrane Hollow Fiber Amicon $H_1P_{100}$.

L'antigène est constituée de la fraction retenue sur ces membranes et lavée par 40 volumes d'eau permutée, filtrée sur membrane 0,22 microns.

Au terme de cette ultrafiltration, la solution est centrifugée 15 mm à 11.500 g puis lyophilisée.

Ce procédé a permis la préparation des antigènes (voir Tableau I) à partir de :

Proteus vulgaris     souche CIP 54160
Proteus mirabilis     souche CIP A 234
Proteus morganii     souche CIP A 231.

Enterobacter cloacae souche CIP 6085
Enterobacter aerogenes souche CIP 6086
Enterobacter aerogenes souche CIP 6023.

Escherichia coli $O_{111}B_4$ souche CIP 52168

Pour la souche Klebsiella pneumoniae CIP 52145, l'antigène a de plus été traité comme indiqué aux exemples 1 et 2 du brevet européen n≠ 49182 (traitement au bromure de cétyltriméthylammonium, centrifugation, éthanol, dialyse, lyophilisation et gel filtration), ou à l'exemple 1 du brevet européen n≠ 0115988.

## TABLEAU 1

| | Rendements en grammes par litre de culture | | |
|---|---|---|---|
| | Souche | Germes | Antigène |
| Proteus vulgaris | CIP 54160 | 10,44 | 0,05 |
| mirabilis | CIP A 234 | 13,6 | 0,23 |
| morganii | CIP A 231 | 4,7 | 0,06 |
| Enterobacter cloaçae | CIP 6085 | 12 | 1,54 |
| Enterobacter aerogenes | CIP 6086 | 12,7 | 0,64 |
| Enterobacter aerogenes | CIP 6023 | 13,1 | 1,8 |
| Escherichia coli $O_{111}B_4$ | CIP 52168 | 4,23 | 0,08 |
| Klebsiella pneumoniae | CIP 52145 | 10 | 0,36 |

II.Immunisation par l'antigène isolé de Klebsiella pneumoniae.

Une souris BALB/C âgée de 8 semaines est immunisée en injectant par voie parentérale au jour zéro et 21, 20 mcg de l'antigène décrit dans le brevet français n≠2.540.136 puis 10 mcg du même antigène à J 35 ; 3 jours après ce dernier rappel, la rate est prélevée.

### III. Préparation des splénocytes

La rate est broyée au polybroyeur et la suspension cellulaire ajustée à $10.10^6$ cellules par millilitre dans du milieu de Dulbecco.

### IV. Préparation du Myelome SP2/0-Ag 14

Une culture de cellules $SP_20$ en phase de croissance est centrifugée, lavée puis reprise dans du milieu de Dulbecco à raison de $8 \times 10^6$ cellules/ml.

### V. Fusion

$15 \times 10^6$ cellules $SP_20$ et $50 \times 10^6$ splénocytes de souris sont centrifugés 5 minutes à 1.100 tr/mn. Le surnageant est éliminé et sur le culot cellulaire, on ajoute goutte à goutte 1 ml de polyéthylèneglycol 1000 à 45% (Merck 9729). Cette opération est réalisée à température ambiante et dure exactement une minute.

Durant la minute suivante, on dissocie le culot avec la pointe d'une pipette. On ajoute ensuite goutte à goutte 2 ml de milieu Dulbecco à raison de 1 ml par minute puis 18 ml de milieu de Dulbecco contenant du sérum de veau foetal (10 % final) à raison de 4,5 ml par minute.

La suspension cellulaire est homogénéisée à la pipette et déposée dans une boîte de Petri de 8 cm de diamètre.

Ces cellules sont cultivées à 37°C dans un incubateur humidifié contenant 8 % de $CO_2$. Après 18 heures de culture, on ajuste la suspension à $2,5 \times 10^5$ cellules/ml dans du Dulbecco contenant 10 % de sérum de veau foetal et on répartit cette suspension cellulaire dans une plaque à 24 trous (COSTAR) à raison de 0,4 ml par cupule. Après 6 heures de culture à 37°C en atmosphère saturée en humidité contenant 8 % de $CO_2$, on ajoute dans chaque godet 1 ml de milieu de sélection dont la formule est la suivante :

Dulbecco (Gibco 041 1965)    90 ml
sérum de veau foetal    10 ml
pénicilline    100 U/ml
streptomycine    100 mcg/ml
glutamine    2 mM
pyruvate    1 mM
hypoxanthine    $5.10^{-5}$ M
azasérine    $10^{-5}$ M

Les cellules sont cultivées dans les mêmes conditions que précédemment. Toutes les semaines, on rajoute 0,2 ml de milieu de sélection frais. Après 2 semaines environ lorsque l'on distingue à l'oeil nu des colonies, on prélève dans chaque cupule du surnageant de culture.

### VI. Détection des hybridomes secrétants.

On détecte les hybridomes secrétant les anticorps dirigés contre la substance ayant servi au dernier rappel de l'immunisation de la souris en soumettant ces surnageants à un test Elisa vis-à-vis de cet antigène. 250 microlitres d'une solution de l'antigène en tampon carbonate/bicarbonate pH 9,6 - 0,05 M sont déposés dans chacune des cupules d'une plaque microelisa 96 trous en immulon (Dynatech. 92430-Marnes la Coquette). Après 18 heures d'incubation à + 4°C en chambre humide, les cupules sont vidées de leur contenu et lavées deux fois par la même solution tampon.

On dépose 300 microlitres de solution de sérumalbumine bovine (BSA Sigma Chemical Co St Louis MO) à 0,1 % dans le tampon carbonate. Après deux heures d'incubation en chambre humide à 37°C, on élimine le liquide et on réalise 3 lavages par le tampon PBS (pH 7,4 ; 0,01 M) contenant 0,5‰ de Tween 20.

Les surnageants d'hybridomes à tester sont dilués dans le PBS Tween et déposés à raison de 200 microlitres par cupule et mis à incuber pendant 18 heures à + 4°C.

Après 4 lavages en PBS Tween des anticorps antiimmunoglobulines de souris, marqués à la phosphatase alcaline sont dilués en PBS Tween contenant 0,1 % de sérum albumine bovine, puis déposés à raison de 200 microlitres par cupule.

Après deux heures d'incubation à température ambiante suivies de 4 lavages, on ajoute 200 microlitres de para nitrophényl phosphate (Sigma) (1 mg/ml en tampon diéthanolamine à pH 9,8). La réaction entre l'enzyme et son substrat est poursuivie une demi-heure à la température ambiante puis bloquée par addition de 50 microlitres d'une solution de soude 3M. Une réaction positive se traduit par une coloration jaune dont on peut apprécier la densité optique à 405 nm.

### VII. Conservation des hybridomes secrétants.

Les hybridomes dont les surnageants contiennent l'anticorps recherché sont mis en culture en milieu Dulbecco contenant 10 % de sérum de veau foetal d'abord en plaques 24 trous puis en boîte de Petri.

Lorsque le nombre de cellules est suffisant, la suspension cellulaire est ajustée à environ $4.10^6$ cellules par ml de sérum de veau foetal contenant 10 % de diméthylsulfoxyde (DMSO). Les hybrides ainsi conditionnés seront conservés dans l'azote liquide.

### VIII. Clonage.

Les hybridomes retenus sont ensuite clonés par la méthode des dilutions limites de manière à sélectionner spécifiquement chacun des clones secrétant lesdits anticorps.

A ce stade, la secrétion des anticorps est mesurée dans les surnageants de culture par le test Elisa cité en VI ci-dessus. Les clones ainsi retenus sont conservés en azote liquide.

IX. Production des anticorps.

a) Production chez la souris.

$6.10^{-5}$ cellules secrétantes sont injectées par voie intrapéritonéale à des souris BALB/C ayant reçu 15 jours auparavant 0,5 ml de Pristane (2,6,10,14-tétraméthyl - pentadécane), 10 à 15 jours après l'injection des cellules clonées, les ascites sont prélevées puis centrifugées à 2000 t/mn.

Le liquide d'ascite ainsi recueilli sert directement aux analyses Elisa (cf. paragraphe VI ci-dessus) et à la mise en évidence de l'activité thérapeutique de ces anticorps dans les états de choc endotoxinique chez la souris (cf. paragraphe XIII).

b) Production in vitro.

La production est également réalisée in vitro dans des flacons ou des fermenteurs.

X. Caractérisation des anticorps monoclonaux.

a) Détermination de l'isotype des anticorps monoclonaux.

L'identification de l'isotype des anticorps monoclonaux présents dans les surnageants de culture a été faite par une méthode de double diffusion en gelose (Ouchterlony) à l'aide d'immuno sérums antichaînes légères ou lourdes d'immunoglobulines de souris, dirigés contre les IgM, IgA, IgG1, IgG$_{2a}$, IgG$_{2b}$, IgG$_3$ (voir tableau II).

b) Spécificité antigénique.

Cette spécificité a été testée vis-à-vis de différentes fractions isolées de Klebsiella pneumoniae à savoir celles décrites dans les brevets français n$\bowtie$ 2.171.907, 2.462.477, 2.490.496 et 2.574.429 ainsi que le lipopolysaccharide de Klebsiella pneumoniae.

Les résultats figurent dans le tableau II (Exemple 1).

XI. Séparation et purification des anticorps monoclonaux.

a) Les anticorps présents dans les surnageants des cultures réalisées in vitro ou les liquides d'ascite sont précipités par le sulfate d'ammonium à 45 % puis dissous dans un tampon Tris, HCl 50 mM. Cette solution est ensuite chromatographiée sur une colonne de DEAE Sephadex préalablement équilibrée avec le même tampon. La fraction non retenue sur la colonne contient les immunoglobulines de l'isotype G.

b) Une purification des anticorps des divers isotypes G peut être obtenue par chromatographie sur Sepharose protéine A ou Ultragel protéine A.

Dans cette méthode, le surnageant de culture cellulaire ou le liquide d'ascite est directement déposé sur une colonne contenant l'immunosorbant dans un tampon phosphate.

Les immunoglobulines de types M et A ne sont pas retenues alors que les IgG restent fixées sur la protéine A, leur élution sélective IgG1, IgG$_{2a}$, IgG$_{2b}$, IgG$_3$ est obtenue en lavant successivement la colonne par les tampons tels que ceux décrits dans la technique de Ey (P.L Ey et al (1978) immunochemistry, 15, p. 429-436).

Les caractéristiques (spécificité, isotype, activité neutralisante du choc endotoxinique des immunoglobulines ainsi purifiées sont analysées comme décrit aux points VI, X et XIII.

XII. Préparation d'immunosorbant et leur utilisation.

Les anticorps purifiés sont fixés sur des billes de Sépharose (Pharmacia) ou d'Ultragel (IBF) préalablement activées par le bromure de cyanogène ou par un époxy. Les antigènes tels que décrits dans les brevets français 2.490.496 et 2.574.429 ou des fragments de ceux-ci sont purifiés par chromatographie d'affinité sur les supports solides ainsi préparés.

XIII. Utilisation thérapeutique de ces anticorps.

Le modèle choisi pour mettre en évidence cet effet est inspiré de celui décrit par Galanos (1979), Proc.Natl.Acad.Sci.USA, 76, n$\bowtie$11,p.5939-5943. Des souris B$_6$D$_2$F$_1$ reçoivent par voie intraveineuse 0,2 ml de liquide d'ascite contenant les anticorps puis 3 heures plus tard par voie intraveineuse l'endotoxine de (Klebsiella pneumoniae) en mélange avec 8 mg de galactosamine. Parallèlement un lot témoin d'animaux reçoit à la place du liquide d'ascite contenant l'anticorps, le même volume d'un liquide d'ascite de souris ayant reçu la cellule SP$_2$/0-Ag14.

Les résultats sont exprimés en nombre d'animaux survivants.

(Voir tableau II (Exemple 1).

## TABLEAU II

### Exemple 1 : Protection dans le choc endotoxinique.

Anticorps monoclonaux dirigés contre Klebsiella pneumoniae.

| | | | NOMBRE DE SOURIS SURVIVANT | |
|---|---|---|---|---|
| CELLULE | ISOTYPE | SPECIFICITE | GROUPE TRAITE | GROUPE TEMOIN |
| 6D5 | $IgG_3$ | | 10/10 | 0/10 |
| 12B6 | $IgG_3$ | Core | 10/10 | 0/10 |

Exemple 2 :
Les animaux sont immunisés par l'antigène de Escherichia coli.

I. Préparation des antigènes (voir exemple 1).

II. Immunisation par l'antigène d'Escherichia Coli.
Une souris BALB/C âgée de 8 semaines est immunisée en injectant par voie parentérale aux jours zéro et 21,50 mcg et 20 mcg respectivement de bactéries tuées par chauffage, au jour 35 une injection des antigènes purifiés LPS-LAP est réalisée à raison de 20 mcg par souris ; 3 jours après ce rappel, la rate est prélevée.

III. Préparation des splénocytes.
La rate est broyée au Polybroyeur et la suspension cellulaire ajustée à $10.10^6$ cellules par millilitre dans du milieu de Dulbecco.

IV. Préparation du Myelome $SP_2$/0-Ag 14
Une culture de cellules $SP_2O$ en phase de croissance est centrifugée, lavée puis reprise dans du milieu de Dulbecco à raison de $8 \times 10^6$ cellules/ml.

V. Fusion
$15 \times 10^6$ cellules $SP_2O$ et $50 \times 10^6$ splénocytes de souris sont centrifugés 5 minutes à 1.100 tr/mn. Le surnageant est éliminé et sur le culot cellulaire, on ajoute goutte à goutte 1 ml de polyéthylèneglycol 1000 à 45% (Merck 9729). Cette opération est réalisée à température ambiante et dure exactement une minute.
Durant la minute suivante, on dissocie le culot avec la pointe d'une pipette. On ajoute ensuite goutte à goutte 2 ml de milieu Dulbecco à raison de 1 ml par minute puis 18 ml de milieu de Dulbecco contenant du sérum de veau foetal (10 % final) à raison de 4,5 ml par minute.
La suspension cellulaire est homogénéisée à la pipette et déposée dans une boîte de Petri de 8 cm de diamètre.
Ces cellules sont cultivées à 37°C dans un incubateur humidifié contenant 8 % de $CO_2$. Après 18 heures de culture, on ajuste la suspension à $2,5 \times 10^5$ cellules/ml dans du Dulbecco contenant 10 % de sérum de veau foetal et on répartit cette suspension cellulaire dans une plaque à 24 trous (COSTAR) à raison de 0,4 ml par cupule. Après 6 heures de culture à 37°C en atmosphère saturée en humidité contenant 8 % de $CO_2$, on ajoute dans chaque godet 1 ml de milieu de sélection dont la formule est la suivante :
Dulbecco (Gibco 320 1965)   90 ml
sérum de veau foetal   10 ml
pénicilline   100 U/ml
streptomycine   100 mcg/ml
glutamine   2 mM
pyruvate   1 mM
hypoxanthine   $5.10^{-5}M$
azasérine   $10^{-5}M$
Les cellules sont cultivées dans les mêmes conditions que précédemment. Toutes les semaines, on rajoute 0,2 ml de milieu de sélection frais. Après 2 semaines environ lorsque l'on distingue à l'oeil nu des colonies, on prélève dans chaque cupule du surnageant de culture.

## VI. Détection des hybridomes secrétants.

On détecte les hybridomes secrétant les anticorps dirigés contre la substance ayant servi au dernier rappel de l'immunisation de la souris en soumettant ces surnageants à un test Elisa vis-à-vis de cet antigène. 250 microlitres d'une solution de l'antigène en tampon carbonate/bicarbonate pH 9,6 - 0,05 M sont déposés dans chacune des cupules d'une plaque microelisa 96 trous en immulon (Dynatech. 92430 Marnes la Coquette). Après 18 heures d'incubation à + 4⋈C en chambre humide, les cupules sont vidées de leur contenu et lavées deux fois par la même solution tampon.

On dépose environ 300 microlitres de solution de sérumalbumine bovine (BSA Sigma Chemical Co St Louis MO) à 0,1 % dans le tampon carbonate. Après deux heures d'incubation en chambre humide à37⋈C, on élimine le liquide et on réalise 3 lavages par le tampon PBS (pH 7,4 ; 0,01 M) contenant 0,5⋈/⋈⋈ de Tween 20.

Les surnageants d'hybridomes à tester sont dilués dans le PBS Tween et déposés à raison de 200 microlitres par cupule et mis à incuber pendant 18 heures à + 4⋈C.

Après 4 lavages en PBS Tween des anticorps antiimmunoglobulines de souris, marquées à la phosphatase alcaline sont dilués en PBS Tween contenant 0,1 % de sérum albumine bovine, puis déposés à raison de 200 microlitres par cupule.

Après deux heures d'incubation à température ambiante suivies de 4 lavages, on ajoute 200 microlitres de para nitrophényl phosphate (Sigma) (1 mg/ml en tampon diéthanolamine à pH 9,8). La réaction entre l'enzyme et son substrat est poursuivie une demi-heure à la température ambiante puis bloquée par addition de 50 microlitres d'une solution de soude 3M. Une réaction positive se traduit par une coloration jaune dont on peut apprécier la densité optique à 405 nm.

## VII. Conservation des hybridomes secrétants.

Les hybridomes dont les surnageants contiennent l'anticorps recherché sont mis en culture en milieu Dulbecco contenant 10 % de sérum de veau foetal d'abord en plaques 24 trous puis en boîte de Petri.

Lorsque le nombre de cellules est suffisant, la suspension cellulaire est ajustée à environ $4.10^6$ cellules par ml de sérum de veau foetal contenant 10 % de diméthylsulfoxyde (DMSO). Les hybrides ainsi conditionnés seront conservés dans l'azote liquide.

## VIII. Clonage.

Les hybridomes retenus sont ensuite clonés par la méthode des dilutions limites de manière à sélectionner spécifiquement chacun des clones secrétant lesdits anticorps.

A ce stade, la secrétion des anticorps est mesurée dans les surnageants de culture par le test Elisa cité en VI ci-dessus.

Les clones ainsi retenus sont conservés en azote liquide.

## IX. Production des anticorps.

a) Production chez la souris.

$6.10^5$ cellules secrétantes sont injectées par voie intrapéritonéale à des souris BALB/C ayant reçu 15 jours auparavant 0,5 ml de Pristane (2,6,10,14-tétraméthyl - pentadécane), 10 à 15 jours après l'injection des cellules clonées, les ascites sont prélevées puis centrifugées à 2000 t/mn.

Le liquide d'ascite ainsi recueilli sert directement aux analyses Elisa (cf. paragraphe VI ci-dessus) et à la mise en évidence de l'activité thérapeutique de ces anticorps dans les états de choc endotoxinique chez la souris (cf. paragraphe XIII).

b) Production in vitro.

La production est également réalisée in vitro dans des flacons ou des fermenteurs.

## X. Caractérisation.

Cette spécifité a été testée en plus par rapport à l'exemple 1 vis-à-vis de LPS d'Escherichia coli de différents sérotypes (DIFCO), de Serratia marcescens (SIGMA), de Klebsiella pneumoniae (LIST), de Klebsiella pneumoniae 01:K2 (CIP 52145), de Pseudomonas aeruginosa 06 (CIP 59-39), 011 (CIP 59-44), 012 (CIP 59-45) préparés par nous-mêmes selon la technique décrite par 0. Westphal (Z. Naturforsch. 1952, 7b, 148-155).

Les résultats du tableau IV montrent que certains des anticorps ($D_4B_5$, $D_5B_5$, $D_5D_2$, $D_6B_3$, $D_9A_2$) reconnaissent les LPS de différents sérotypes d'Escherichia coli et que d'autres ($D_6B_4$, $D_{11}A_5$, $B_7B_3$) sont en plus capables de reconnaître des LPS d'autres entérobactéries comme ceux de Serratia ou de Klebsiella (tableau IVA). L'anticorps $B_7B_3$ réagit fortement avec les LPS de Pseudomonas aeruginosa de sérotypes 06, 011, 012 ainsi qu'avec les germes entiers des mêmes sérotypes, ainsi que du sérotype M (P. aeruginosa ATCC 21636) (tableau IV B).

## XI. Séparation et purification des anticorps monoclonaux.

a) Les anticorps présents dans les surnageants des cultures réalisées in vitro ou les liquides d'ascite sont précipités par le sulfate d'ammonium à 45 % puis dissous dans un tampon Tris, HCl 50 mM. Cette solution est ensuite chromatographiée sur une colonne de DEAE Sephadex préalablement équilibrée avec le même tampon. La fraction non retenue sur la colonne contient les immunoglobulines de l'isotype G.

b) Une purification des anticorps des divers isotypes G peut être obtenue par chromatographie sur Sepharose protéïne A ou Ultragel protéïne A.

Dans cette méthode, le surnageant de culture cellulaire ou le liquide d'ascite est directement déposé sur une colonne contenant l'immunosorbant dans un tampon phosphate.

Les immunoglobulines de types M et A ne sont pas retenues alors que les IgG restent fixées sur la protéïne A, leur élution sélective $IgG_1$, $IgG_{2a}$, $IgG_{2b}$, $IgG_3$ est obtenue en lavant successivement la colonne par les tampons tels que ceux décrits dans la technique de Ey (P.L. Ey et al.(1978) Immunochemistry, 15, p. 429-436).

Les caractéristiques (spécificité, isotype, activité neutralisante du choc endotoxinique des immunoglobulines ainsi purifiées sont analysées comme décrit aux points VI, X et XIII.

XII. Préparation d'immunosorbant et leur utilisation.

Les anticorps purifiés sont fixés sur des billes de Sépharose (Pharmacia) ou d'Ultragel (IBF) préalablement activées par le bromure de cyanogène ou par un époxy. Les antigènes tels que décrits dans les brevets français 2.490.496 et 2.574.429 ou des fragments de ceux-ci sont purifiés par chromatographie d'affinité sur les supports solides ainsi préparés.

XIII. Utilisation thérapeutique de ces anticorps.

Le modèle choisi pour mettre en évidence cet effet est inspiré de celui décrit par Galanos (1979),Proc.Natl.Acad.Sci.USA,76, n=11, p.5939-5943. Des souris $B_6D_2F_1$ reçoivent par voie intraveineuse 0,2 ml de liquide d'ascite contenant les anticorps puis 3heures plus tard par voie intraveineuse l'endotoxine de E.C. en mélange avec 8 mg de galactosamine. Parallèlement un lot témoin d'animaux reçoit à la place du liquide d'ascite contenant l'anticorps, le même volume d'un liquide d'ascite de souris ayant reçu la cellule $SP_2/0$-Ag14.

Les résultats sont exprimés en nombre d'animaux survivants. (Voir tableau III (Exemple 2).

## TABLEAU III

### Exemple 2 : Protection dans le choc endotoxinique.

#### Anticorps monoclonaux dirigés contre Escherichia Coli.

|  |  |  | NOMBRE DE SOURIS SURVIVANT | |
| --- | --- | --- | --- | --- |
| CELLULE | ISOTYPE | SPECIFICITE | GROUPE TRAITE | GROUPE TEMOIN |
| $D_6B_4$ | $IgG_3$ | rough LPS | 10/10 | 1/10. |

La cellule $D_6B_4$ secrète une immunoglobuline protectrice dans le choc endotoxinique.

TABLEAU IV A

0 271 379

## HYBRIDOMES ANTI-LPS DE E.COLI
### REACTIVITE EN ELISA VIS A VIS DE DIVERS LPS

| | LPS E.COLI J5 RC 2169 | LPS K.p RC 2149 | LPS K.p LIST | LPS E.COLI DIFCO 0111 B4 | LPS E.COLI DIFCO 055 B5 | LPS E.COLI DIFCO 0127 B8 | LPS E.COLI DIFCO 0128 B12 | LPS SERRATIA SIGMA L. 6136 |
|---|---|---|---|---|---|---|---|---|
| B7B3 | +++ | − | +/− | − | ++ | − | +++ | +++ |
| D4B5 | +++ | − | − | +++ | +/− | − | ++ | − |
| D5B5 | +/− | − | − | + | +++ | +++ | + | − |
| D5D2 | +++ | − | − | +++ | ++ | − | +/− | − |
| D6B3 | +++ | − | + | +++ | +++ | +++ | +++ | − |
| D6B4 | +++ | +/− | + | +++ | +++ | +++ | +++ | +/− |
| D9A2 | +++ | − | − | +/− | +/− | − | + | − |
| D11A5 | +++ | − | +/− | +++ | +++ | +++ | +++ | +/− |

Exemple 3:
Les animaux sont immunisés par les antigènes de Proteus ou d'Enterobacter (tableau I) (voir les exemples 1 et 2 pour les points : I, II, III, IV, V, VI, VII, VIII, IX, XI, XII).

X. Caractérisation:
La spécifité des anticorps a été testée vis-à-vis des antigènes préparés à partir des souches de Proteus ou d'Enterobacter. l'anticorps $E_{22}B_6$ reconnaît les antigènes préparés à partir de 3 souches de Proteus (tableau V), l'anticorps $H_7A_2$ reconnaît les antigènes préparés à partir des 3 souches d'Enterobacter (tableau VI).

### HIBRIDOME $B_7B_3$
### Réactivité en Elisa vis à vis de Pseudomonas aeruginosa

|  | | LPS | |
|---|---|---|---|
| P. aeruginosa réf. sérotype | CIP 59.39 06 | CIP 59.44 011 | CIP 59.45 012 |
| Anticorps $B_7B_3$ | +++ | +++ | +++ |

|  | | GERMES ENTIERS | | |
|---|---|---|---|---|
| P. aeruginosa réf. sérotype | CIP 59.39 06 | CIP 59.44 011 | CIP 59.45 012 | ATCC 21636 M |
| Anticorps $B_7B_3$ | +++ | +++ | +++ | +++ |

## TABLEAU V

### HIBRIDOMES ANTI-PROTEUS

Réactivité en ELISA vis à vis des antigènes de Proteus

| Antigènes obtenus à partir des souches | P. mirabilis CIP A 234 | P. vulgaris CIP 54.160 | P. morganii CIP A 231 |
|---|---|---|---|
| Anticorps $E_{22}B_6$ | +++ | +++ | +++ |

## TABLEAU VI

### HIBRIDOMES ANTI-ENTEROBACTER

Réactivité en ELISA vis à vis des antigènes d'Enterobacter

| Antigènes obtenus à partir des souches | E. aerogenes CIP 60.23 | E. aerogenes CIP 60.86 | E. cloacae CIP 60.85 |
|---|---|---|---|
| Anticorps $H_7A_2$ | +++ | +++ | +++ |

## I. ANTICORPS MONOCLONAUX ANTI E.COLI.

Protection contre l'infection expérimentale.

### 1) Matériel et méthode.

Des groupes de 10 souris femelles âgées de 5-6 semaines OFI (IFFA-CREDO) ont reçu une injection intrapéritonéale de 1 ml d'une solution de chlorure de sodium à 0,9% renfermant 5% de silice (Kielselguhr Merck référence 8119), 90 minutes avant d'être infectées.

Deux souches d'Escherichia Coli, 0111:B4 (C.I.P. 52167) et 02:KI:H4 (C.I.P. 54-52) sont incubées pendant 18 heures à 37⊨C dans un milieu Tryptocaséine-soja (Institut Mérieux). Le mélange est alors dilué par addition d'une solution de chlorure de sodium 0,9% à la concentration requise et 0,5 ml de cette suspension sont injectés dans la cavité péritonéale. Le nombre de bactéries est déterminé.

### 2) Résultats.

Deux anticorps $D_6B_3$ et $D_6B_4$ asministrés 3 heures avant l'infection par un E. Coli 0111:B4 protégeant significativement les souris (tableau VII).

Une estimation de la dose efficace a été faite en administrant les anticorps $D_6B_4$ à des doses variant de 10 mcg à 1 mg par souris.

Une protection a encore été constatée à une dose de 50 mcg par souris (tableau VIII).

L'injection de l'anticorps $D_6B_4$ une heure après l'infection a également donné une protection significative bien qu'elle soit moins efficace que le traitement préventif (tableau IX).

Dans le cas d'une infection hétérogène avec E. Coli 02 une augmentation assez nette de la période de survie a été observée pour les animaux ayant reçu l'anticorps $D_6B_3$ 3 heures avant l'infection.

Infection expérimentale avec E. Coli 0111:B4

Effet du traitement préventif par des anticorps monoclonaux

### TABLEAU VII

| Prétraitement | | E. Coli 0111:B4 | Mortalité | |
|---|---|---|---|---|
| Anticorps contrôle | dose (mg) par souris | infection bactérie par souris | le 15e jour morts/total | Signification |
| PBS (b) | | 3,2 x 10 | 10/10 | |
| N3 19 | 1 | 3,2 x 10 | 10/10 | |
| D6B4 | 1 | 3,2 x 10 | 1/10 | 0,0001 |

(a) méthode de Fisher

(b) anticorps monoclonal Antinéomycine B utilisé comme contrôle négatif.

Des groupes de 10 souris ont reçu une injection I.V. d'anticorps précipités au sulfate d'ammonium 3 heures avant l'infection par E. Coli 0111:B4 selon le modèle de sensibilisation par la silice. Une solution apyrogène PBS est utilisée comme témoin. La mortalité des animaux est relevée tous les jours pendant 14 jours. La méthode de Fisher est utilisée dans la détermination de la signification.

## TABLEAU VIII

| Prétraitement | | E. Coli 0111:B4 infection bactérie par souris | Mortalité le 15e jour morts/total | Signification |
|---|---|---|---|---|
| Anticorps contrôle | dose (mg) par souris | | | |
| **Test 1** | | | | |
| PBS | | $3,3 \times 10^7$ | 10/10 | |
| | | $1,7 \times 10^7$ | 10/10 | |
| N319 (b) | 1 | $3,3 \times 10^7$ | 10/10 | |
| | | $1,7 \times 10^7$ | 10/10 | |
| D6B4 | 1 | $3,3 \times 10^7$ | 1/10 | 0,0001 |
| | | $1,7 \times 10^7$ | 0/10 | $< 0,0001$ |
| | 0,2 | $3,3 \times 10^7$ | 0/10 | $< 0,0001$ |
| | | $1,7 \times 10^7$ | 2/10 | 0,0007 |
| | 0,1 | $3,3 \times 10^7$ | 1/10 | 0,0001 |
| | | $1,7 \times 10^7$ | 1/10 | 0,0001 |
| | 0,05 | $3,3 \times 10^7$ | 5/10 | 0,032 |
| | | $1,7 \times 10^7$ | 2/10 | 0,0007 |
| **Test 2** | | | | |
| PBS | | $1,8 \times 10^7$ | 10/10 | |
| D6B4 | 0,1 | $1,8 \times 10^7$ | 3/10 | 0,003 |
| | 0,05 | $1,8 \times 10^7$ | 5/10 | 0,032 |
| | 0,02 | $1,8 \times 10^7$ | 6/10 | NS (c) |
| | 0,01 | $1,8 \times 10^7$ | 10/10 | |

(a) méthode de Fisher

(b) anticorps monoclonal Antinéomycine B utilisé comme contrôle négatif.

(c) NS = non significatif

Des groupes de 10 souris ont reçu une injection I.V. de différentes doses d'anticorps précipités au sulfate d'ammonium 3 heures avant l'infection par E. Coli 0111:B4. Le protocole est identique à celui figurant dans le tableau VII.

Infection expérimentale avec E. Coli 0111:B4

Effet d'un traitement curatif par des anticorps monoclonaux

## TABLEAU IX

| Prétraitement | | E. Coli 0111:B4 | Mortalité | |
|---|---|---|---|---|
| Anticorps contrôle | dose (mg) par souris | infection bactérie par souris | le 15e jour morts/total | Signification |
| PBS | | $3,5 \times 10^7$ | 10/10 | |
| | | $1,8 \times 10^7$ | 10/10 | |
| N319 (b) | 1 | $3,5 \times 10^7$ | 10/10 | |
| | | $1,8 \times 10^7$ | 10/10 | |
| D6B4 | 1 | $3,5 \times 10^7$ | 6/10 | NS (c) |
| | | $1,8 \times 10^7$ | 3/10 | 0,003 |
| | 0,2 | $3,5 \times 10^7$ | 6/10 | NS |
| | | $1,8 \times 10^7$ | 3/10 | 0,003 |

(a) méthode de Fisher

(b) anticorps monoclonal Antinéomycine B utilisé comme contrôle négatif.

(c) NS = non significatif

Des groupes de 10 souris ont reçu une injection I.V. d'anticorps précipités au sulfate d'ammonium (aux doses indiquées) 1 heure après l'infection par E. Coli 0111:B4. Le protocole est identique à celui figurant dans le tableau VII.

Infection expérimentale avec E. Coli 02:K1

Effet du traitement préventif par des anticorps monoclonaux $D_6B_3$

## TABLEAU X

| Prétraitement Anticorps contrôle | dose (mg) par souris | E. Coli 02:K1 infection bactérie par souris | Durée de survie moy. en jours | Signification |
|---|---|---|---|---|
| PBS | | $2,5 \times 10^{7}$ | 4,9 | |
| D6B3 | 0,6 | $2,5 \times 10^{7}$ | 8,4 | 0,019 |

Des groupes de 8 souris (SWISS) ont reçu une injection I.V. d'anticorps précipités au sulfate d'ammonium 3 heures avant l'infection par E. Coli 02:K1 selon le modèle de sensibilisation par la silice. Une solution apyrogène PBS est utilisée comme témoin. La mortalité des animaux est relevée tous les jours pendant 13 jours. La durée moyenne de survie est comparés à celle des témoins (méthode de Mann et Withney).

## II. ANTICORPS MONOCLONAUX ANTI KP.

Protection contre l'infection expérimentale.

Des groupes de 10 souris femelles âgées de 5-6 semaines OFl (IFFA-CREDO) sont utilisées. Une souche de K. pneumoniae 01:K2 est mise à incuber pendant 18 heures à 37⊨C sur un milieu d'agar. Le mélange est alors dilué par addition d'une solution de chlorure de sodium 0,9% à la concentration requise et 0,5 ml de cette suspension sont injectés dans la cavité péritonéale.

Traitement par des anticorps monoclonaux.

Les anticorps monoclonaux sont purifiés par 2 précipitations au sulfate d'ammonium à 45% de saturation suivie d'une dialyse vis à vis d'une solution apyrogène PBS. La concentration en anticorps monoclonaux est déterminée par la technique d'immunodiffusion radiale décrite par Mancini.

Infection expérimentale par K pneumoniae 01:K2

Effet du traitement préventif par des anticorps monoclonaux

# 0 271 379

## TABLEAU XI

| Prétraitement | | K. pneumoniae 01:K2 | Mortalité | |
|---|---|---|---|---|
| Anticorps contrôle | dose (mg) par souris | infection bactérie par souris | le 14e jour morts/total | Signification |
| PBS | | $1,6 \times 10^3$ | 10/10 | |
| N319 (b) | 0,6 | $1,6 \times 10^3$ | 10/10 | NS |
| 12B6 | 0,6 | $1,6 \times 10^3$ | 3/10 | 0,003 |

(a) méthode de Fisher

(b) anticorps monoclonal Antinéomycine B utilisé comme contrôle négatif.

Des groupes de 10 souris ont reçu une injection I.V. d'anticorps précipités au sulfate d'ammonium 3 heures avant l'infection par K. pneumoniae 01:K2. Une solution apyrogène PBS est utilisée comme témoin. La mortalité des animaux est relevée tous les jours pendant 14 jours. La méthode de Fisher est utilisée dans la détermination de la signification.

## Revendications

1) Procédé de préparation d'anticorps monoclonaux de mammifères dirigés contre la partie lipide A-core de l'endotoxine des bactéries gram (-) de différents genres, caractérisé en ce que l'antigène servant à l'immunisation des cellules immunocompétentes est un lipopolysaccharide complet de bactérie gram (-) dont la partie lipide A-core est extériorisée.

2) Procédé selon la revendication 1, caractérisé en ce que le lipopolysaccharide complet provient de bactéries des genres Escherichia, Klebsiella, Pseudomonas, Enterobacter, Bacteroïdes, Serratia ou Proteus.

3) Procédé selon la revendication 2, caractérisé en ce que le lipopolysaccharide complet provient de Klebsiella pneumoniae.

4) Procédé selon la revendication 2, caractérisé en ce que le lipopolysaccharide complet provient de Escherichia coli.

5) Procédé selon la revendication 1, 2,3 ou 4, caractérisé en ce que le lipopolysaccharide se présente sous une structure lipopolysaccharide protéines associées au lipide A.

6) Anticorps monoclonaux obtenus par le procédé selon l'une des revendications 1 à 4.

7) Anticorps monoclonaux murins selon la revendication 6.

8) Anticorps monoclonaux humains selon la revendication 6.

9) Hybridomes obtenus lors de la mise en oeuvre du procédé selon les revendications 1 à 5.

10) Hybridomes producteurs d'anticorps monoclonaux choisis dans le groupe $D_6B_4$,6 $D_5$, 12 $B_6$, $B_7B_3$, $E_{22}B_6$ et $H_7A_2$.

11) Médicaments caractérisés en ce qu'ils sont constitués par les anticorps monoclonaux selon l'une des revendications 6 à 8.

12) Compositions pharmaceutiques ou diagnostiques renfermant les anticorps monoclonaux selon l'une des revendications 6 à 8.

13) Compositions destinées à la purification de produits renfermant la partie antigénique aspécifique des bactéries gram (-) caractérisées en ce qu'elles utilisent les anticorps monoclonaux selon les revendications 6 à 8.

17

Revendications pour l'Etat contractant suivant : GR

1) Procédé de préparation d'anticorps monoclonaux de mammifères dirigés contre la partie lipide A-core de l'endotoxine des bactéries gram (-) de différents genres, caractérisé en ce que l'antigène servant à l'immunisation des cellules immunocompétentes est un lipopolysaccharide complet de bactérie gram (-) dont la partie lipide A-core est extériorisée.

2) Procédé selon la revendication 1, caractérisé en ce que le lipopolysaccharide complet provient de bactéries des genres Escherichia, Klebsiella, Pseudomonas, Enterobacter, Bacteroïdes, Serratia ou Proteus.

3) Procédé selon la revendication 2, caractérisé en ce que le lipopolysaccharide complet provient de Klebsiella pneumoniae.

4) Procédé selon la revendication 2, caractérisé en ce que le lipopolysaccharide complet provient de Escherichia coli.

5) Procédé selon la revendication 1, 2,3 ou 4, caractérisé en ce que le lipopolysaccharide se présente sous une structure lipopolysaccharide protéines associées au lipide A.

6) Procédé selon l'une quelconque des revendications 1 à 5, caractérisé en ce que l'on prépare des anticorps monoclonaux murins.

7) Procédé selon l'une quelconque des revendications 1 à 5, caractérisé en ce que l'on prépare des anticorps monoclonaux humains.

8) Hybridomes obtenus lors de la mise en oeuvre du procédé selon les revendications 1 à 5.

9) Hybridomes producteurs d'anticorps monoclonaux choisis dans le groupe $D_6B_4$, $6D_5$, 12 $B_6$, $B_7B_3$, $E_{22}B_6$ et $H_7A_2$

Revendications pour l'Etat contractant suivant : ES

1) Procédé de préparation d'anticorps monoclonaux de mammifères dirigés contre la partie lipide A-core de l'endotoxine des bactéries gram (-) de différents genres, caractérisé en ce que l'antigène servant à l'immunisation des cellules immunocompétentes est un lipopolysaccharide complet de bactérie gram (-) dont la partie lipide A-core est extériorisée.

2) Procédé selon la revendication 1, caractérisé en ce que le lipopolysaccharide complet provient de bactéries des genres Escherichia, Klebsiella, Pseudomonas, Enterobacter, Bacteroïdes, Serratia ou Proteus.

3) Procédé selon la revendication 2, caractérisé en ce que le lipopolysaccharide complet provient de Klebsiella pneumoniae.

4) Procédé selon la revendication 2, caractérisé en ce que le lipopolysaccharide complet provient de Escherichia coli.

5) Procédé selon la revendication 1, 2,3 ou 4, caractérisé en ce que le lipopolysaccharide se présente sous une structure lipopolysaccharide protéines associées au lipide A.

6) Procédé selon l'une quelconque des revendications 1 à 5, caractérisé en ce que l'on prépare des anticorps monoclonaux murins.

7) Procédé selon l'une quelconque des revendications 1 à 5, caractérisé en ce que l'on prépare des anticorps monoclonaux humains.

8) Procédé selon l'une quelconque des revendications 1 à 7, caractérisé en ce que l'on prépare des hybridomes producteurs d'anticorps monoclonaux tels que définis ci-dessus.

9) Procédé selon l'une quelconque des revendications 1 à 7, caractérisé en ce que l'on prépare hybridomes producteurs d'anticorps monoclonaux choisis dans le groupe $D_6B_4$, $6D_5$, 12 B6, $B_7B_3$, $E_{22}B_6$ et $H_7A_2$.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | EUROPEAN JOURNAL OF IMMUNOLOGY, vol. 12, 1982, pages 797-803, Verlag Chemie GmbH, Weinheim, DE; J.R. HIERNAUX et al.: "Study of the idiotypy of lipopolysaccharide-specific polyclonal and monoclonal antibodies" * Page 797, colonne 2, lignes 34-36; page 798, colonne 1, lignes 19-48; page 799, colonne 1, lignes 17-42 * | 1-10 | C 12 P   21/00 C 12 N    5/00 C 12 N   15/00 A 61 K   39/40 // (C 12 P   21/00 C 12 R    1:91 ) |
| Y | IDEM | 11-13 | |
| Y | WO-A-8 404 458  (POLLACK, MATTHEW) * Page 6, lignes 8-24; page 10, lignes 23-36; page 11, lignes 1-11; page 15, lignes 11-25 * | 11-13 | |
| A | INFECTION AND IMMUNITY, vol. 46, no. 3, décembre 1984, pages 677-681, American Society of Microbiology, US; M.J. NELLES et al.: "Mouse monoclonal antibodies reactive with J5 lipopolysaccharide exhibit extensive serological cross-reactivity with a variety of gram-negative bacteria" * Page 677, colonne 1, lignes 21-29; page 681, colonne 1, lignes 3-7 * | 1-13 | DOMAINES TECHNIQUES RECHERCHES (Int. Cl.4) A 61 K C 12 P |
| A | EP-A-0 183 876  (THE BOARD OF TRUSTEES OF THE LELAND STANFORD JUNIOR UNIVERSITY) | | |
| A | WO-A-8 501 659  (CENTOCOR INC.) | | |

Le présent rapport a été établi pour toutes les revendications

| Lieu de la recherche | Date d'achèvement de la recherche | Examinateur |
|---|---|---|
| LA HAYE | 18-02-1988 | FERNANDEZ Y BRANAS F.J. |